# NEUE EUROPÄISCHE PATENTSCHRIFT

(11) **EP 2 406 070 B2**
(45) Veröffentlichungstag und Bekanntmachung des Hinweises auf die Entscheidung über den Einspruch: **06.12.2017**
(45) Hinweis auf die Patenterteilung: 27.08.2014
(21) Anmeldenummer: 10706951.0
(22) Anmeldetag: 03.03.2010
(51) Int. Cl.: B32B 5/04, A61F 13/15, B32B 3/22, B32B 27/12, B32B 37/14, A61F 13/49, B32B 5/02, B32B 5/06, B32B 37/12, B32B 3/04, B32B 3/18

(54) **ELASTISCHES LAMINAT, INSBESONDERE FÜR ELASTISCHE WINDELVERSCHLUSSELEMENTE**
ELASTIC LAMINATE, IN PARTICULAR FOR ELASTIC DIAPER FASTENING ELEMENTS
STRATIFIÉ ÉLASTIQUE, NOTAMMENT POUR ÉLÉMENTS DE FERMETURE ÉLASTIQUES DE COUCHE-CULOTTE

(30) Priorität: 11.03.2009 EP 09003523
(43) Veröffentlichungstag der Anmeldung: 18.01.2012
(73) Patentinhaber: Mondi Gronau GmbH, 48599 Gronau (DE)
(72) Erfinder: BALDAUF, Georg, 48366 Laer (DE); SCHÖNBECK, Marcus, 33775 Versmold (DE)
(74) Vertreter: Lorenz, Bernd Ingo Thaddeus
(86) Internationale Anmeldenummer: PCT/EP2010/001293
(87) Internationale Veröffentlichungsnummer: WO 2010/102742

(56) Entgegenhaltungen:
- EP-A- 1 342 562
- EP-A- 1 342 563
- EP-A- 1 541 106
- EP-B- 1 261 479
- WO-A-01/54900
- WO-A-98/54389
- WO-A-03/007864
- WO-A-95//19258
- WO-A-08//028106
- WO-A-2004/060673
- WO-A-2007/095104
- US-A- 5 769 993
- US-A-2008/0 051 748
- US-A1- 2004 121 687
- US-A1- 2008 207 071
- US-B- 6 726 983

## Beschreibung

Die Erfindung betrifft ein elastisches Laminat, insbesondere für elastische Windelverschlusselemente, mit Außenschichten aus Nonwoven und einer zumindest bereichsweise zwischen den Außenschichten einkaschierten elastischen Folie, wobei zumindest eine der beiden Außenschichten aus einem in Querrichtung dehnbaren, durch Wasserstrahlen verfestigten Vliesstoff besteht, der im Bereich der elastischen Folie in zumindest einer Achsrichtung verstreckt ist. Das Laminat wird als Materialbahn gefertigt, aus der Elemente ausgestanzt werden können, die quer zur Maschinenrichtung des Laminats elastisch sind. Aus dem elastischen Laminat können insbesondere elastische Frontpartien für Wegwerfwindeln, die auch als Windelohren bezeichnet werden, gefertigt werden.

Ein Laminat mit den beschriebenen Merkmalen ist aus EP 1 921 192 A1 bekannt. Die Außenschichten bestehen aus einem Faserflor aus Stapelfasern oder Endlosfasern, der durch eine Wasserstrahlbehandlung verfestigt worden ist. Der durch Wasserstrahlen verfestigte Vliesstoff ist mit geringer Kraft um bis zu 100 % dehnbar. Bei einer weiteren Dehnung nimmt die Dehnkraft progressiv zu, bis bei einer Dehnung von etwa 200 % die Dehngrenze erreicht ist. Die Dehngrenze ist als Begrenzung deutlich wahrnehmbar.

Ein Verstrecken des Laminats nach dem Laminierungsprozess in Querrichtung wird in 2004/0121687 A1 offenbart.

Ausgehend von dem beschriebenen Stand der Technik liegt der Erfindung die Aufgabe zugrunde, die Dehnungseigenschaften des elastischen Laminats weiter zu verbessern.

Zur Lösung dieser Aufgabe lehrt die Erfindung ein Laminat nach Anspruch 1.

Der wasserstrahlverfestigte Vliesstoff (spunlace nonwoven), der zumindest eine der beiden Außenschichten bildet, enthält schlaufenförmige Faserstrukturen, die sich infolge der Wasserstrahlbehandlung gebildet haben. Aufgrund dieser schlaufenförmigen Strukturen, die für eine Wasserstrahlverfestigung charakteristisch sind, lässt sich das Nonwoven mit einer geringen Kraft um bis zu 100 % dehnen. Bei einer weitergehenden Dehnung nimmt die Dehnungskraft progressiv zu. Überraschenderweise zeigt sich nun, dass die Dehnungscharakteristik eines aus einer elastischen Folie und einem wasserstrahlverfestigten Vliesstoff bestehenden Kaschierverbundes durch eine Verstreckung des Vliesstoffes günstig beeinflusst werden kann. Je nach Art und Grad der mechanischen Vorverstreckung lassen sich unterschiedliche Effekte erzielen, die einzeln oder in Kombination die Dehnungseigenschaften des Laminats in Querrichtung vorteilhaft verändern.

Eine Verstreckung des Laminats in Querrichtung erfolgt zweckmäßig durch ein Ringrollverfahren, bei dem das aus äußeren Nonwovenschichten und einer einkaschierten elastischen Folie bestehende Laminat durch eine Profilwalzenanordnung durchgeführt wird. Die Profilwalzenanordnung weist zumindest zwei ineinandergreifende Profilwalzen auf, die alternierend aus Profilwalzenabschnitten mit großen und kleinen Durchmessern zusammengesetzt sind. In der Profilwalzenanordnung wird das Laminat quer zur Laufrichtung lokal überdehnt. Durch die Eingrifftiefe der Profilwalzen ist der Verstreckungsgrad einstellbar. Bereits durch eine geringe Verstreckung des Laminats in Querrichtung kann die Dehnungscharakteristik des Laminats vorteilhaft beeinflusst werden.

Gemäß der Erfindung wird das Laminat durch eine Dehnung, die kleiner ist als eine durch die Bindungsstruktur des wasserstrahlverfestigten Vliesstoffes vorgegebene Dehngrenze, in Querrichtung verstreckt, wobei die Verstreckung erfindungsgemäß mit einem Dehnwert zwischen 100 % und 200 % ausgeführt ist. Bei einer Verstreckung mit einem geringen Verstreckungsgrad bleibt die Bindungsstruktur des wasserstrahlvernetzten Nonwovens weitgehend erhalten. Gleichwohl wird beobachtet, dass der progressive Dehnungskraftanstieg, der für einen wasserstrahlvernetzten Vliesstoff bei einer Dehnung von mehr als 100 % charakteristisch ist, deutlich abgeschwächt ist. Im Ergebnis zeichnet sich das in der beschriebenen Weise verstreckte und dadurch vorbehandelte Laminat dadurch aus, dass die Dehnungskraft über einen weiten Dehnungsbereich relativ konstant bleibt. Da die Bindungsstruktur des Nonwovens bei einer Verstreckung von weniger als 200 % weitgehend erhalten bleibt, entspricht die Dehngrenze im Wesentlichen dem Wert eines nicht verstreckten Materials.

Erfindungsgemäß ist vorgesehen, dass eine Außenschicht aus einem wasserstrahlverfestigten Vliesstoff besteht, während die zweite Außenschicht aus einem Vlies mit einem mehrschichtigen Aufbau des Typs SMS (spun-melt-spun) besteht. Die zweite Außenschicht kann thermisch oder chemisch durch Zugabe von Harzen verfestigt sein.

Zwischen den beiden Außenschichten aus Nonwoven kann eine elastische Folie flächig einkaschiert sein, die sich über die gesamte Bahnbreite erstreckt. Eine bevorzugte Ausführung der Erfindung sieht allerdings vor, dass zwischen den Außenschichten elastische Folienstreifen einkaschiert sind, wobei die Folienstreifen nebeneinander angeordnet und in Querrichtung zueinander beabstandet sind. Zwischen den elastischen Folienstreifen kann eine nicht dehnbare Verstärkungsschicht aus einem Polymer, einer Folie oder einem Spunlaid-Vliesstoff angeordnet sein, die den Abstand zwischen den elastischen Folienstreifen überbrückt und die Folienstreifen randseitig überlappt. Das auf diese Weise gebildete elastische Laminat weist elastische und nicht elastische Abschnitte auf.

Die Schichten des elastischen Laminats können thermisch durch punktförmige Ultraschallverschweißungen oder durch eine Klebeverbindung verbunden sein. Eine bevorzugte Ausführung der Erfindung sieht vor, dass die Außenschichten aus Nonwoven mit der elastischen Folie verklebt sind, wobei der Klebstoff vollflächig, streifenförmig, gitterförmig oder punktförmig auf die zu verbindenden Flächen aufgetragen ist.

## Patentansprüche

1. Elastisches Laminat, insbesondere für elastische Windelverschlusselemente, mit Außenschichten aus Nonwoven und einer zumindest bereichsweise zwischen den Außenschichten einkaschierten elastischen Folie, wobei zumindest eine der beiden Außenschichten aus einem in Querrichtung dehnbaren, durch Wasserstrahl verfestigten Vliesstoff besteht, der im Bereich der elastischen Folie in zumindest einer Achsrichtung verstreckt ist, **dadurch gekennzeichnet, dass** das Laminat durch eine Dehnung mit einem Dehnwert zwischen 100 % und 200 % in Querrichtung verstreckt ist, wobei die Dehnung kleiner ist als eine durch die Bindungsstruktur des Wasserstrahl verfestigten Vliesstoffes vorgegebene Dehngrenze, wobei die Schichten des elastischen Laminates durch punktförmige Ultraschallverschweißungen oder durch eine Klebstoffverbindung verbunden sind und wobei die zweite Außenschicht aus einem Vlies mit einem mehrschichtigen Aufbau des Typs SMS (spun-melt-spun) besteht.

2. Elastisches Laminat nach Anspruch 1, **dadurch gekennzeichnet, dass** zwischen den Außenschichten elastische Folienstreifen einkaschiert sind, wobei die Folienstreifen nebeneinander angeordnet und in Querrichtung zueinander beabstandet sind.

3. Elastisches Laminat nach Anspruch 2, **dadurch gekennzeichnet, dass** zwischen den elastischen Folienstreifen eine nicht dehnbare Verstärkungsschicht aus einem Polymer, einer Folie oder einem Spunlaid-Vliesstoff angeordnet ist, die den Abstand zwischen den elastischen Folienstreifen überbrückt und die Folienstreifen randseitig überlappt.

4. Elastisches Laminat nach einem der Ansprüche 1 bis 3, **dadurch gekennzeichnet, dass** die Außenschichten aus Nonwoven mit der elastischen Folie verklebt sind, wobei der Klebstoff vollflächig, streifenförmig, gitterförmig oder punktförmig auf die zu verbindenden Flächen aufgetragen ist.

## Claims

1. An elastic laminate, in particular for elastic nappy closure elements, with outer layers of non-woven and an elastic film laminated-in between the outer layers at least in sections, wherein at least one of the two outer layers consists of a fleece fabric expandable in transverse direction and solidified by a water jet, which has been stretched in the region of the elastic film in at least one axial direction, **characterised in that** the laminate has been stretched in transverse direction by expanding it with an expansion value between 100% and 200%, wherein the expansion is less than an expansion limit defined by the bonding structure of the water jet solidified fleece fabric, wherein the layers of the elastic laminate are connected by spot-like ultrasound welds or by an adhesive connection and wherein the second outer layer consists of a fleece having a multi-layer construction of type SMS (spun-melt-spun).

2. The elastic laminate according to claim 1, **characterised in that** elastic film strips have been laminated-in between the outer layers, wherein the film strips are arranged adjacent to each other and spaced apart from each other in transverse direction.

3. The elastic laminate according to claim 2, **characterised in that** a non-expandable reinforcement layer from a polymer, a film or a spunlaid fleece material is arranged between the elastic film strips, which layer bridges the distance between the elastic film strips and overlaps the film strips at the edges.

4. The elastic laminate according to one of claims 1 to 3, **characterised in that in that** the outer layers from a non-woven are glued to the elastic film, wherein the adhesive is applied to the surfaces to be connected either all-over, in strips, in a grid pattern or spot-like.

## Revendications

1. Laminé élastique, notamment pour des éléments élastiques de fermeture de couche, avec des couches extérieures en non-tissé et un film élastique contrecollé au moins par zones entre les couches extérieures, au moins l'une des deux couches extérieures étant composée d'une étoffe nappée extensible en direction transversale, solidifiée par jet d'eau, qui dans la zone du film élastique est allongée dans au moins une direction axiale, **caractérisé en ce que** le laminé est allongé dans la direction transversale par un allongement d'une valeur d'allongement comprise entre 100 % et 200 %, l'allongement étant inférieur à une limite d'allongement prédéfinie par l'étoffe nappée solidifiée par la structure de liaison du jet d'eau, les couches du laminé élastique étant reliées par des soudures ponctuelles aux ultrasons ou par une liaison par agent adhésif et la deuxième couche extérieure étant composée d'un voile d'une structure multicouches de type SMS (spun-melt-spun).

2. Laminé élastique selon la revendication 1, **caractérisé en ce qu'**entre les couches extérieures sont contrecollés des rubans de film élastiques, les rubans de film étant placés côte à côte et étant écartés les uns des autres dans la direction transversale.

3. Laminé élastique selon la revendication 2, **caractérisé en ce qu'**entre les rubans de film élastiques est placée une couche de renfort non extensible en un polymère, un film ou un voile de filaments, qui recouvre l'écart entre les rubans de film élastiques et chevauche les rubans de film du côté marginal.

4. Laminé élastique selon l'une quelconque des revendications 1 à 3, **caractérisé en ce que** les couches extérieures en non-tissé sont collées avec le film élastique, l'agent adhésif étant appliqué à pleine surface, en forme de bandes, en forme de grille ou en forme de points sur les surfaces qui doivent être reliées.
